# EUROPÄISCHE PATENTSCHRIFT

(11) **EP 1 261 301 B1**
(45) Veröffentlichungstag und Bekanntmachung des Hinweises auf die Patenterteilung: **15.09.2004**
(21) Anmeldenummer: 00907398.2
(22) Anmeldetag: 10.03.2000
(51) Int. Cl.: A61F 2/38

(54) **KOMPONENTE FÜR EINE GELENKENDOPROTHESE**
COMPONENT FOR A JOINT ENDOPROSTHESIS
COMPOSANT POUR UNE ENDOPROTHESE D'ARTICULATION

(43) Veröffentlichungstag der Anmeldung: 04.12.2002
(73) Patentinhaber: Biomet Merck GmbH, 3216 Ried b. Kerzers (CH)
(72) Erfinder: HERZOG, Roland, CH-4437 Waldenburg (CH); CORTELLESSA, Maurizio, CH-3400 Burgdorf (CH); KOLLER, Markus, CH-4123 Allschwil (CH); ZIRNGIBL, Nicolas, CH-4104 Oberwil (CH)
(74) Vertreter: Lusuardi, Werther Giovanni, Dr.
(86) Internationale Anmeldenummer: PCT/CH2000/000140
(87) Internationale Veröffentlichungsnummer: WO 2001/066046

(56) Entgegenhaltungen:
- EP-A- 0 553 585
- GB-A- 2 245 175
- US-A- 5 413 608

## Beschreibung

Die Erfindung bezieht sich auf eine Komponente für eine Kniegelenkendoprothese gemäss dem Oberbegriff des Patentanspruchs 1.

Insbesondere bei Kniegelenkprothesen bestehen solche Komponenten aus einem metallischen, in der Tibia zu verankernden Teil, einem aus Kunststoff bestehenden, mit dem metallischen Prothesenteil verbundenen Teil und einem metallischen im Femur zu verankernden Teil. Die Verbindung zwischen dem Tibiateil und dem Kunststoffteil kann fix oder verschiebbar sein. Durch diese ebene Verschiebbarkeit zwischen den beiden Komponenten wird eine Gleitfläche der Kniegelenkprothese gebildet.

Eine solche Gelenkprothese, insbesondere Kniegelenkprothese ist beispielsweise aus der EP 0 519 873 BÄHLER bekannt. Diese bekannte Kniegelenkprothese besteht im wesentlichen aus einem ersten Prothesenteil mit Mitteln zur Verankerung im Femur und zwei Kondylen, einem zweiten Prothesenteil mit einer Gleitfläche und Mitteln zur Verankerung in der Tibia, einem Zwischenteil, welches auf der Gleitfläche verschiebbar ist, und einem Kupplungsorgan. Das Kupplungsorgan ist mittels eines Zapfens, welcher in einer Bohrung im Tibiateil aufgenommen wird, frei drehbar im Tibiateil gelagert. Zudem besitzt dieses Kupplungsorgan eine gekrümmte Schwalbenschwanzführung, welche mit einer dazu korrespondierenden Schwalbenschwanzführung am Zwischenteil im Eingriff steht. Diese Schwalbenschwanzführung sorgt für eine geführte Bewegung des Zwischenteils auf der Gleitfläche. Der Bewegungsablauf dieser bekannten Kniegelenkprothese entspricht daher weitgehend dem physiologischen Bewegungsablauf des natürlichen Gelenks. Zur Artikulationsbewegung dienen die Kondylen sowie die dazu korrespondierenden Lagerflächen auf dem Zwischenteil. Die Translationsbewegung, z. B. von anterior nach posterior wird durch die vom Kupplungsorgan geführte Verschiebung des Zwischenteils auf der Gleitfläche ermöglicht, während der Drehzapfen am Kupplungsorgan die Rotation um die Tibialängsachse ermöglicht.

Bei anderen bekannten Ausgestaltungen von Kniegelenkprothesen ist auch eine Dislokation des Kunststoffteiles in der vertikalen möglich.

Nachteilig an dieser bekannten Kniegelenkprothese ist, dass das Zwischenteil bezüglich der Tibialängsachse frei drehbar mit dem Tibiateil der Prothese verbunden ist. Kräfte, welche eine Verdrehung von Femur und Tibia um die Längsachsen dieser Knochen verursachen müssen in ihrem vollen Umfang von den Bändern aufgenommen werden. Bei zu schwachen Bändern ist eine Rotationsdislokation des Kunststoffteiles möglich.

Hier will die Erfindung Abhilfe schaffen. Der Erfindung liegt die Aufgabe zugrunde, die Rotationsbewegung, welche zwischen Zwischenteil und Tibiateil möglich sein muss, in einem bestimmten Drehwinkelbereich zu begrenzen und eine Sicherung gegen ein Abheben des Zwischenteils vom Tibiateil zu gewährleisten.

Die Erfindung löst die gestellte Aufgabe mit einer Komponente für eine Kniegelenkendoprothese, welche die Merkmale des Anspruchs 1 aufweist.

Weitere vorteilhafte Ausgestaltungen der Erfindung sind in den abhängigen Ansprüchen gekennzeichnet.

Die erfindungsgemässe Komponente, welche zusammen mit einem nicht zur erfindungsgemässen Komponente gehörenden Femurteil eine Gelenkendoprothese, insbesondere eine Kniegelenkendoprothese bildet umfasst im wesentlichen ein metallisches Verankerungselement, welches auf seiner Unterseite einen Verankerungszapfen zur Befestigung in einem Knochen und eine quer zum Verankerungszapfen verlaufende ebene Oberseite aufweist, eine aus Kunststoff bestehenden Zwischenplatte, welche eine obere Fläche mit Gleitflächen für den Femurteil der Endoprothese und eine ebene untere Fläche aufweist. Die untere Fläche der Zwischenplatte ist auf der Oberseite des Verankerungselementes verschiebbar und der Verankerungszapfen verläuft nach einer Implantation in einem Knochen im wesentlichen parallel zu dessen Längsachse. Das Verankerungselement enthält mittig senkrecht zu seiner Oberseite einen Lagerzapfen mit einer Längsachse, welcher in einem dazu korrespondierenden Loch in der Zwischenplatte um die Längsachse rotierbar aufgenommen wird. Ferner durchdringt parallel zur Oberseite des Verankerungselementes ein Loch mit einer Zentralachse den Lagerzapfen und parallel zur unteren Fläche der Zwischenplatte ist ein weiteres Loch mit einer zur Zentralachse fluchtenden Längsachse in der Zwischenplatte angebracht. Ein ebenfalls zur erfindungsgemässen Komponente gehörendes Verbindungselement, welches als Stift oder Schrauben ausführbar ist, ist durch die beiden Löcher bringbar. Zudem ist eines der beiden Löcher mindestens auf einem Teil seiner Länge als Langloch ausgestaltet, so dass die Zwischenplatte innerhalb eines definierten Winkelbereiches um den Lagerzapfen drehbar ist. Das Langloch hat einen Querschnitt mit einer zur Oberseite des Verankerungselement parallelen Breite b, welche entlang der Zentralachse auch variabel sein kann, beispielsweise gegen das Zentrum des Lagerzapfens verjüngend, und einer Höhe h.

Das Verankerungselement und die Zwischenplatte weisen einen senkrecht zur Längsachse stehenden, im wesentlichen ovalen Querschnitt auf. Die Zentralachse der zur Aufnahme des Stiftes bestimmten Löcher im Lagerzapfen und in der Zwischenplatte verläuft dabei im wesentlichen parallel zur kürzeren Achse des ovalen Querschnittes. Die Gleitflächen sind bezüglich dieser Zentralachse einander gegenüberliegend angeordnet.

In der bevorzugten Ausführungsform der erfindungsgemässen Komponente weisen der Stift einen Durchmesser d und eines der Löcher im Querschnitt eine Höhe h und eine maximale Breite b auf, wobei h ≥ d ist und die maximale Breite b grösser als die Höhe h und so bemessen ist, dass bei zusammengesetzter Komponente die Bewegung des Stiftes im Loch eine Drehung der Zwischenplatte um die Längsachse bis zu einem maximalen Drehwinkel von 40° zulässt.

In anderen Ausführungsformen der erfindungsgemässen Komponente kann dieser maximale Drehwinkel kleiner sein und vorzugsweise 10° sein.

Die Ausgestaltung der zur Aufnahme des Stiftes bestimmten Löcher im Lagerzapfen und in der Zwischenplatte kann verschieden gestaltet sein:
- Das Loch im Lagerzapfen verjüngt sich von den Durchdringungsstellen des Loches mit der Mantelfläche des Lagerzapfens zum Zentrum des Lagerzapfens hin während das Loch in der Zwischenplatte kreiszylindrisch ist;
- Das Loch im Lagerzapfen ist als zylindrisches Langloch ausgebildet während das Loch in der Zwischenplatte kreiszylindrisch ist; oder
- Das Loch in der Zwischenplatte ist als zylindrisches Langloch ausgebildet während das Loch im Lagerzapfen kreiszylindrisch ist.

Zur Ausgestaltung einer Stiftverbindung, welche bei zusammengesetzter Komponente senkrecht zur Oberseite des Verankerungselementes ein Spiel zwischen dem Verankerungselement und der Zwischenplatte zulässt, kann die Höhe h zwischen d ≤ h ≤ 2 d, vorzugsweise zwischen d ≤ h ≤ 1,2 d betragen. Dies ermöglicht eine Bewegung der Zwischenplatte auf dem Verankerungselement innerhalb einer bestimmten Rotation, ohne dass sich der Stift und der Lagerzapfen berühren auch wenn die Unterseite der Zwischenplatte durch langjährigen Gebrauch abgenutzt wurde.

In weiteren Ausführungsformen der erfindungsgemässen Komponente sind der Durchmesser d des Stiftes, die Höhe h und die minimale Breite bₘᵢₙ des Lochquerschnittes so bemessen, dass der Stift in demjenigen Loch, welches als Langloch ausgebildet ist, auch parallel zur Oberseite des Verankerungselementes Spiel erhält.

Das Loch in der Zwischenplatte, welches zur Aufnahme des Lagerzapfens dient, kann kreiszylindrisch sein zur rein rotativen Lagerung des Lagerzapfens oder als Langloch zur rotativen und verschiebbaren Lagerung des Lagerzapfens ausgebildet werden. Dabei kann der Lagerzapfen prismatisch, zylindrisch oder kreiszylindrisch ausgebildet sein.

Der Stift ist gegen axiale Verschiebung gesichert. Dies kann dadurch erreicht werden, dass
- Der Stift ist in das Loch mit kreisförmigem Querschnitt eingepresst;
- Das Loch in der Zwischenplatte einseitig geschlossen ist;
- Der Stift mit Zähnen versehen ist, wodurch der Stift in demjenigen Loch, welches einen kreisförmigen Querschnitt aufweist, verklemmt;
- Der Stift mit Widerhaken versehen ist, wodurch der Stift in demjenigen Loch, welches einen kreisförmigen Querschnitt aufweist, verklemmt;
- Der Stift weist auf seinem Umfang mindestens eine planare Einfräsung auf während das Loch eine oder mehrere entsprechende Ausbuchtungen aufweist; und
- Das Loch ist mit mindestens einer Einfräsung versehen während der Stift entsprechende Ausbuchtungen aufweist.

Zudem kann der Stift an einem Ende, vorzugsweise an seinem anterioren Ende. eine Bohrung mit Innengewinde umfassen, welche zur Befestigung eines Werkzeuges zum Einbringen oder Entfernen des Stiftes aus der Komponente dient.

In den verschiedenen Ausführungsformen der erfindungsgemässen Komponente kann der Lagerzapfen einen Durchmesser D zwischen 8 bis 15 mm aufweisen während Stiftdurchmesser d zwischen 2 bis 6 mm günstig sind.

Die durch die Erfindung erreichten Vorteile sind im wesentlichen darin zu sehen, dass dank der erfindungsgemässen Komponente die Drehbewegung um die ungefähr in der Verlängerung der Knochenachse liegende Drehachse des Zwischenelementes um das Tibiateil innerhalb eines bestimmten Winkelbereiches begrenzt ist und eine Sicherung gegen Abheben des Zwischenteils vom Tibiateil gewährt ist, ohne dabei die Gelenkfunktion zu beeinträchtigen.

Das Femurteil und das Tibiateil können nach verschiedenen bekannten Operationstechniken implantiert werden. Der Stift wird normalerweise nach der Implantation des Femurteils, des Tibiateils und des Kunststoffteils von anterior nach posterior in die dafür vorgesehene Öffnung im Kunststoffteil eingebracht.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 einen Schnitt durch eine Ausführungsform des Verankerungselementes der erfindungsgemässen Kniegelenkendoprothese;
Fig. 2 eine Ansicht der in Fig. 1 dargestellten Ausführungsform der Zwischenplatte der erfindungsgemässen Kniegelenkendoprothese;
Fig. 3 eine Aufsicht auf eine Ausführungsform der Zwischenplatte der erfindungsgemässen Kniegelenkprothese;
Fig. 4 eine Aufsicht auf eine Ausführungsform des Lagerzapfens der erfindungsgemässen Kniegelenkendoprothese; und
Fig. 5 eine Ansicht von vorne auf eine Ausführungsform des Lagerzapfens der erfindungsgemässen Kniegelenkendoprothese.

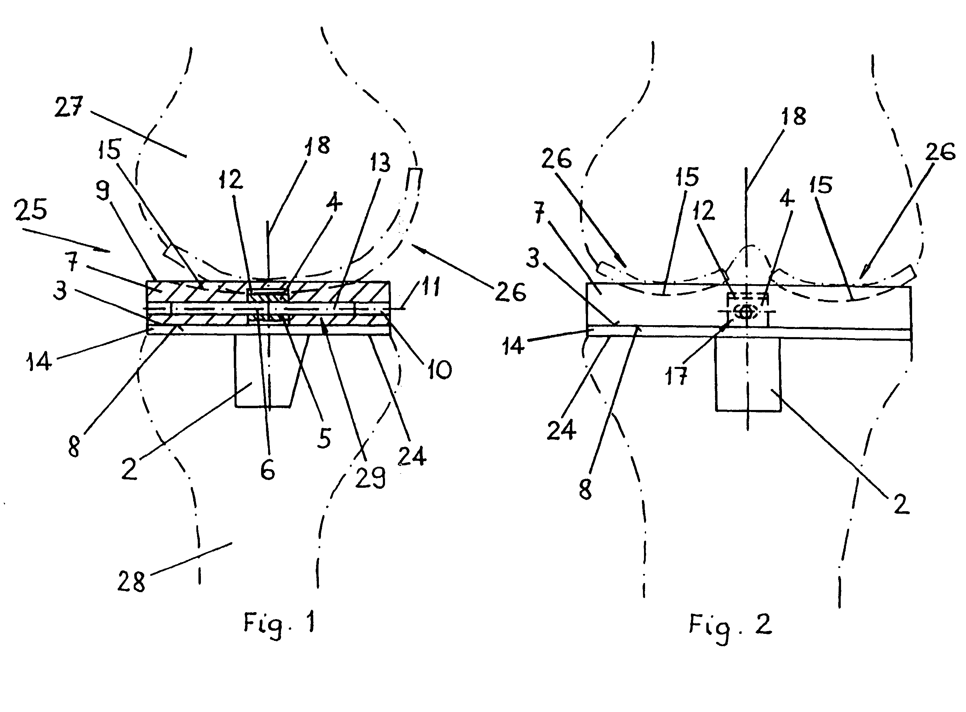

Die Fig. 1 und 2 zeigen die bevorzugte Ausführungsform der erfindungsgemässen Komponente zusammen mit einem Femurteil für eine Endogelenkprothese am Beispiel einer Kniegelenkendoprothese. Die Komponente umfasst ein metallisches Verankerungselement 14, welches einen Verankerungszapfen 2 zur Befestigung in einem Knochen und eine quer zum Verankerungszapfen 2 verlaufende ebene Oberseite 3 aufweist, und eine aus Kunststoff bestehende Zwischenplatte 7 mit einer oberen, die Gleitflächen der Endoprothese enthaltenden Fläche 9 und einer unteren. auf der Oberseite 3 verschiebbaren Fläche 8. Der Verankerungszapfen 2 ist so am Verankerungselement 14 angeordnet, dass nach einer Implantation des Verankerungselementes 14 in einem Knochen der Verankerungszapfen 2 im wesentlichen parallel zu dessen Längsachse verläuft während die ebene Oberseite 3 orthogonal zur Knochenlängsachse verläuft. Senkrecht zur Oberseite 3 und im wesentlichen mittig zu deren Fläche umfasst das Verbindungselement 14 einen Lagerzapfen 4 mit einer Längsachse 18, welcher in einer dazu korrespondierenden, im wesentlichen mittig in der unteren Fläche 8 angeordneten Bohrung 12 in der Zwischenplatte 7 spielfrei um die Längsachse 18 rotierbar aufgenommen wird. Parallel zur Oberseite 3 durchdringt ein Loch 5 mit einer Zentralachse 6 den Lagerzapfen 4 und ebenfalls parallel zur unteren Fläche 8 durchdringt ein weiteres Loch 10 mit einer zur Zenralachse 6 fluchtenden Längsachse 11 die Zwischenplatte 7. Ferner umfasst die erfindungsgemässe Komponente einen Stift 13, welcher durch die beiden Löcher 5;10 bringbar ist, wobei die Abmessungen des Stiftes 13 und der Löcher 5;10 so bemessen sind, dass bei eingefügtem Stift 13 die Oberseite 3 des Verankerungselementes 14 und die untere Fläche 8 der Zwischenplatte 7 spielfrei aufeinander aufliegen. Das Durchgangsloch 5 ist als Langloch 17 ausgebildet, wobei die senkrecht zur Längachse 18 stehende Breite b des Langloches 17 so gewählt ist, dass bei festgelegten Durchmessern des Stiftes 13 und des Loches 5 die Zwischenplatte 7 innerhalb eines Winkelbereiches von + 40° bis - 40° um die Längsachse 18 des Lagerzapfens 4 drehbar ist.

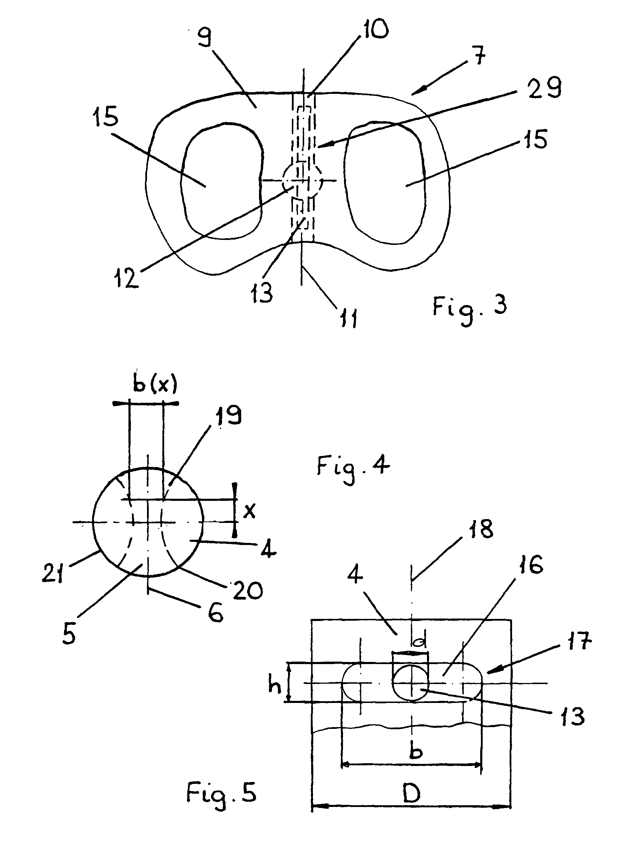

Fig. 3 zeigt eine Ansicht von oben auf eine Ausführungsform der Zwischenplatte 7 der erfindungsgemässen Komponente. Auf der oberen Fläche 9 sind dazu konkave Gleitflächen 15 zur gleitbaren Aufnahme der Femurkomponente 26 angeordnet, wobei die Gleitflächen 15 im wesentlichen symmetrisch zu einer Ebene sind, welche die Zentralachse 11 des Loches 10 schneidet und orthogonal zur unteren Fläche 8 (Fig. 2) steht. Das Loch 10 ist in dieser Ausführungsform als Langloch ausgebildet.

Fig. 4 zeigt eine Aufsicht auf eine Ausführungsform des Lagerzapfens 4 der erfindungsgemässen Komponente, worin das Loch 5 nicht zylindrisch ausgebildet ist. Die Breite b(x) des Loches 5 vergrössert sich mit dem Abstand x vom Zentrum des Lagerzapfens 4. Die maximale Breite b(xₘₐₓ) an den Durchdringungstellen 19;20 des Loches 5 mit der Mantelfläche 21 des Lagerzapfens 4 ist dabei so bemessen, dass sie bei festgelegten Durchmessern d und D eine Drehung der Zwischenplatte 7 innerhalb eines Winkelbereiches von + 40° bis - 40° um die Längsachse 18 des Lagerzapfens 4 zulässt. Für die minimale Breite b(x=0) des Loches 5 gilt b(x=0) ≥ d, so dass der Stift 13 die Wand des Loches 5 im normalen Gebrauch der Komponente nicht berührt.

In Fig. 5 ist eine Ansicht auf eine Ausführungsführungsform des Lagerzapfens 4 frontal zum Langloch 17 dargestellt. Der Lagerzapfen 4 weist den Durchmesser D auf. Das Langloch 17 hat einen ovalen Querschnitt 16 mit der Höhe h parallel zur Längsachse 18 des Lagerzapfens 4 und der Breite b orthogonal zur Längsachse 18 des Lagerzapfens 4. Für die Höhe h gilt in dieser Ausführungsform der erfindungsgemässen Komponente h ≈ 1,1 d, wobei d der Durchmesser des Stiftes 13 ist. Damit wird erreicht, dass der Stift 13 im Loch 10 parallel zur Längsachse 18 des Lagerzapfens 4 Spiel erhält. wodurch die Zwischenplatte 7 gegenüber dem Verankerungselement 14 auch in zur Längsachse 18 paralleler Richtung innerhalb dieses Spiels bewegbar wird. Die Breite b ist so gewählt, dass bei festgelegten Durchmessern d und D die Zwischenplatte 7 innerhalb eines Winkelbereiches von + 40° bis - 40° um die Längsachse 18 des Lagerzapfens 4 drehbar ist. Beispielsweise ergibt sich für die Durchmesser d = 3 mm und D = 12 mm eine Breite b von 9,8 mm.

## Patentansprüche

1. Komponente für eine Gelenkendoprothese welche insbesondere zusammen mit einer Femurkomponente (26) eine Kniegelenkendoprothese bildet, mit
A) einem Verankerungselement (14), welches eine Unterseite (24), eine im wesentlichen dazu parallele, ebene Oberseite (3) und einen im wesentlichen senkrecht auf der Unterseite (24) stehenden Verankerungszapfen (2) zur Befestigung in einem Knochen umfasst, wobei der Verankerungszapfen (2) zu einer zur Längsachse eines Knochens im wesentlichen parallelen Implantation bestimmt ist;
B) einer Zwischenplatte (7), welche eine obere Fläche (9) mit Gleitflächen (15) zur gleitbaren Aufnahme der Femurkomponente (26) und eine ebene untere Fläche (8) umfasst und die untere Fläche (8) auf der Oberseite (3) des Verankerungselementes (14) verschiebbar ist; wobei
C) das Verankerungselement (14) senkrecht zur Oberseite (3) und im wesentllichen mittig auf der Oberseite (3) angeordnet einen Lagerzapfen (4) mit einer Längsachse (18) umfasst, welcher in einem dazu korrespondierenden, im wesentlichen mittig in der unteren Fläche (8) angeordneten, in die Zwischenplatte (7) eindringenden Loch (12) um die Längsachse (18) rotierbar aufgenommen wird;
**dadurch gekennzeichnet, dass**
D) der Lagerzapfen (4) ein Loch (5) mit einer Zentralachse (6) umfasst, welche im wesentlichen parallel zur Oberseite (3) verläuft;
E) in der Zwischenplatte (7) ein weiteres Loch (10) mit einer Längsachse (11) angebracht ist, welches bei zusammengesetzter Komponente zur Zentralachse (6) koaxial ausrichtbar ist;
F) die Komponente zusätzlich ein Verbindungselement (29) umfasst, welches in die beiden Löcher (5;10) bringbar ist; und
G) mindestens eines der beiden Löcher (5;10) mindestens auf einem Teil seiner Länge als Langloch (17) mit einer Höhe h parallel zur Längsachse (18) und einer Breite b senkrecht dazu ausgestaltet ist, so dass die Zwischenplatte (7) bei zusammengesetzter Komponente innerhalb eines definierten Winkelbereiches um den Lagerzapfen (4) drehbar ist.

2. Komponente nach Anspruch 1 **dadurch gekennzeichnet, dass** der Stift (13) einen Durchmesser d aufweist, h ≥ d ist und die maximale Breite b des Langloches (17) so bemessen ist, dass bei zusammengesetzter Komponente die Bewegung des Stiftes (13) im Loch (5;10) eine Drehung der Zwischenplatte (7) um die Längsachse (18) bis zu einem maximalen Drehwinkel von ± 40° zulässt.

3. Komponente nach Anspruch 2, **dadurch gekennzeichnet, dass** der maximale Drehwinkel ± 10° beträgt.

4. Komponente nach einem der Ansprüche 1 bis 3, **dadurch gekennzeichnet, dass** b > h ist.

5. Komponente nach einem der Ansprüche 2 bis 4, **dadurch gekennzeichnet, dass** d ≤ h ≤ 2 d ist.

6. Komponente nach einem der Ansprüche 1 bis 5, **dadurch gekennzeichnet, dass** das Verankerungselement (14) und die Zwischenplatte (7) senkrecht zur Längsachse (18) stehend, einen im wesentlichen ovalen Querschnitt aufweisen, die Zentralachse (6) im wesentlichen parallel zur kürzeren Achse des ovalen Querschnittes angeordnet ist und die Gleitflächen (15) bezüglich der Zentralachse (6) einander gegenüberliegen.

7. Komponente nach einem der Ansprüche 1 bis 6, **dadurch gekennzeichnet, dass** der Lagerzapfen (4) eine Mantelfläche (21) aufweist, das Langloch (17) diese Mantelfläche (21) an den Durchdringungsstellen (19;20) durchdringt und sich die Breite b des Langloches (17) im Lagerzapfen (4) von den Durchdringungsstellen (19;20) zum Zentrum des Lagerzapfens (4) hin verjüngt.

8. Komponente nach einem der Ansprüche 1 bis 7, **dadurch gekennzeichnet, dass** das Loch (5) im Lagerzapfen (4) als zylindrisches oder prismatisches Langloch (17) ausgebildet ist.

9. Komponente nach einem der Ansprüche 1 bis 7, **dadurch gekennzeichnet, dass** das Loch (10) in der Zwischenplatte (7) als zylindrisches oder prismatisches Langloch (17) ausgebildet ist.

10. Komponente nach einem der Ansprüche 1 bis 9, **dadurch gekennzeichnet, dass** das Langloch (17) eine minimale Breite bₘᵢₙ aufweist und der Durchmesser d des Stiftes (13), die Höhe h und die minimale Breite bₘᵢₙ des Langloches (17) so bemessen sind, dass der Stift (13) in demjenigen Loch (5;10), welches als Langloch (17) ausgebildet ist, Spiel erhält.

11. Komponente nach einem der Ansprüche 1 bis 10, **dadurch gekennzeichnet, dass** das Loch (12) ein Langloch ist.

12. Komponente nach einem der Ansprüche 1 bis 11, **dadurch gekennzeichnet, dass** der Lagerzapfen (4) prismatisch ist.

13. Komponente nach einem der Ansprüche 1 bis 11, **dadurch gekennzeichnet, dass** der Lagerzapfen (4) zylindrisch ist.

14. Komponente nach Anspruch 13, **dadurch gekennzeichnet, dass** der Lagerzapfen (4) kreiszylindrisch ist.

15. Komponente nach einem der Ansprüche 1 bis 14, **dadurch gekennzeichnet, dass** das Verbindungselement (29) eine Schraube ist.

16. Komponente nach einem der Ansprüche 1 bis 14, **dadurch gekennzeichnet, dass** das Verbindungselement (29) ein Stift (13) ist

17. Komponente nach Anspruch 16, **dadurch gekennzeichnet, dass** der Stift (13) gegen axiale Verschiebung gesichert ist.

18. Komponente nach Anspruch 17, **dadurch gekennzeichnet, dass** das Loch (10) in der Zwischenplatte (7) einseitig geschlossen ist.

19. Komponente nach Anspruch 17 oder 18, **dadurch gekennzeichnet, dass** der Stift (13) an seinem Umfang mit Zähnen versehen ist.

20. Komponente nach Anspruch 17 oder 18, **dadurch gekennzeichnet, dass** der Stift (13) mit Widerhaken versehen ist.

21. Komponente nach Anspruch 17 oder 18, **dadurch gekennzeichnet, dass** der Stift (13) mit mindestens einer planaren Einfräsung versehen ist, und das Loch (10) entsprechende Einbuchtungen aufweist.

22. Komponente nach Anspruch 17 oder 18, **dadurch gekennzeichnet, dass** das Loch (10) mit mindestens einer Einfräsung versehen ist, und der Stift (13) entsprechende Einbuchtungen aufweist.

23. Komponente nach einem der Ansprüche 16 bis 22, **dadurch gekennzeichnet, dass** der Stift (13) an einem Ende eine Bohrung mit Innengewinde zur Befestigung eines Werkzeuges umfasst.

24. Komponente nach Anspruch 23, **dadurch gekennzeichnet, dass** eine Bohrung mit Innengewinde am anterioren Ende des Stiftes (13) angebracht ist.

25. Komponente nach einem der Ansprüche 14 bis 24, **dadurch gekennzeichnet, dass** der Lagerzapfen (4) einen Durchmesser D zwischen 8 bis 15 mm aufweist.

26. Komponente nach einem der Ansprüche 16 bis 25, **dadurch gekennzeichnet, dass** der Stift (13) einen Durchmesser d zwischen 2 bis 6 mm aufweist.

27. Komponente nach einem der Ansprüche 1 bis 26, **dadurch gekennzeichnet, dass** das Loch (5) den Lagerzapfen (4) durchdringt.

## Claims

1. A joint endoprosthesis component which in particular in conjunction with a femur component (26) constitutes a knee-joint endoprosthesis, comprising
(A) an anchoring element (14) comprising an underside (24), an upper side (3) running substantially parallel thereto, and an anchoring stud (2) which is configured substantially perpendicular on the underside (24) and is used for affixation in a bone, the anchoring stud (2) being used in an implantation which is substantially parallel to a longitudinal axis of the a bone,
(B) a shim (7) comprising an upper surface (9) fitted with slide faces (15) to slidably receive the femur component (26) and a plane lower surface (8) which is displaceable on the top side (3) of the anchoring element (14); where
(C) the anchoring element (14) comprises a bearing pivot (4) which is configured perpendicularly to and substantially centrally on the top side (3) and which exhibits a longitudinal axis (18) and which is received so as to be rotatable about the longitudinal axis (18) in a matching, substantially central hole (12) configured in the lower surface (8) and penetrating the shim (7);
**characterized in that**
(D) The bearing pivot (4) comprises a hole (5) having a center axis (6) running substantially parallel to the top side (3),
(E) A further hole (10) having a longitudinal axis (11) is configured in the shim (7) may be coaxially aligned with the center axis (6) when the component is in its assembled state,
(F) The component moreover includes a coupling element (29) which may be moved into both holes (5; 10), and
(G) At least one of the two holes (5; 10) shall be designed at least along part of its length as an oblong hole (17) having a height h parallel to the longitudinal axis (18) and a width b perpendicular thereto in such manner that when the component is in its assembled state it shall be rotatable about the bearing pivot (4) within a defined angular range.

2. Component as claimed in claim 1, **characterized in that** the pin (13) has a diameter d such that h ≥ d and the maximum width b of the oblong hole (17) is selected in such a way that when the component is in its assembled state the motion of the pin (13) in the hole (5; 10) allows a rotation of the shim (7) about the longitudinal axis (18) up to a maximum angle of rotation of ± 40°.

3. Component as claimed in claim 2, **characterized in that** the maximum angle of rotation is ± 10°.

4. Component as claimed in one of claims 1 through 3, **characterized in that** b > h.

5. Component as claimed in one of claims 2 through 4, **characterized in that** d ≤ h ≤ 2d.

6. Component as claimed in one of claims 1 through 5, **characterized in that** the anchoring element (14) and the shim (7) perpendicular to the longitudinal axis (18) exhibit a substantially oval cross-section, **in that** the center axis (6) is configured substantially parallel to the shorter axis of the oval cross-section and **in that** the slide faces (15) are mutually opposite relative to the center axis (6).

7. Component as claimed in one of claims 1 through 6, **characterized in that** the bearing pivot (4) comprises a lateral surface (21), **in that** the oblong hole (17) penetrates said surface (21) at the penetration sites (19; 20) and **in that** the width b of the oblong hole (17) in the bearing pivot (4) tapers from the penetration sites (19; 20) toward the center of the bearing pivot (4).

8. Component as claimed in one of claims 1 through 7, **characterized in that** the hole (5) in the bearing pivot (4) is a cylindrical or prismatic oblong hole (17).

9. Component as claimed in one of claims 1 through 7, **characterized in that** the hole (10) in the shim (7) is a cylindrical or prismatic oblong hole (17).

10. Component as claimed in one of claims 1 through 9, **characterized in that** the oblong hole (17) exhibits a minimum width bₘᵢₙ and **in that** the diameter d of the pin (13), the height h and the minimum width bₘᵢₙ of the oblong hole (17) are selected in such manner that the pin (13) is received with play **in that** hole (5; 10) which is designed as the oblong hole (17).

11. Component as claimed in one of claims 1 through 10, **characterized in that** the hole (12) is an oblong hole.

12. Component as claimed in one of claims 1 through 11, **characterized in that** the bearing pivot (4) is prismatic.

13. Component as claimed in one of claims 1 through 11, **characterized in that** the bearing pivot (4) is cylindrical.

14. Component as claimed in claim 13, **characterized in that** the bearing pivot (4) is circular-cylindrical.

15. Component as claimed in one of claims 1 through 14, **characterized in that** the coupling element (29) is a screw.

16. Component as claimed in one of claims 1 through 14, **characterized in that** the coupling element (29) is a pin (13).

17. Component as claimed in claim 16, **characterized in that** the pin (13) is secured against axial displacement.

18. Component as claimed in claim 17, **characterized in that** the hole (10) is unilaterally closed in the shim (7).

19. Component as claimed in either of claims 17 and 18, **characterized in that** the pin (13) is fitted with teeth at its circumference.

20. Component as claimed in either of claims 17 and 18, **characterized in that** the pin (13) is fitted with barbs.

21. Component as claimed in either of claims 17 and 18, **characterized in that** the pin (13) is fitted with at least one planar milling and **in that** the hole (10) comprises matching indentations.

22. Component as claimed in either of claims 17 and 18, **characterized in that** the hole (10) is fitted with at least one milling and the pin (13) comprises matching indentations.

23. Component as claimed in one of claims 16 through 22, **characterized in that** the pin (13) is fitted at one end with a threaded borehole to affix a tool.

24. Component as claimed in claim 23, **characterized in that** a threaded borehole is configured at the anterior end of the pin (13).

25. Component as claimed in one of claims 14 through 24, **characterized in that** the bearing pivot (4) has a diameter D between 8 and 15 mm

26. Component as claimed in one of claims 16 through 25, **characterized in that** the pin (13) has a diameter d between 2 and 6 mm.

27. Component as claimed in one of claims I through 26, **characterized in that** the hole (5) penetrates the bearing pivot (4).

## Revendications

1. Composant pour une endoprothèse articulaire qui forme, notamment ensemble avec un composant fémoral (26), une endoprothèse de l'articulation du genou, ledit composant comportant
A) un élément d'ancrage (14) qui comprend une face inférieure (24), une face supérieure plane (3) essentiellement parallèle à celle-ci et un tourillon d'ancrage (2) s'étendant essentiellement perpendiculairement à la face inférieure (24) et destiné à être fixé dans un os, le tourillon d'ancrage (2) étant destiné à être implanté de manière essentiellement parallèle à l'axe longitudinal d'un os;
B) une plaque intermédiaire (7) qui comprend une surface supérieure (9) avec des surfaces de glissement (15) destinées à recevoir le composant fémoral (26) de manière à ce qu'il puisse glisser et une surface inférieure plane (8) et ladite surface inférieure (8) peut coulisser sur la face supérieure (3) de l'élément d'ancrage (14);
C) l'élément d'ancrage (14) comprenant, perpendiculairement à la face supérieure (3) et essentiellement au centre de la face supérieure (3), un tourillon (4) avec un axe longitudinal (18) qui est logé dans un trou (12) correspondant, disposé essentiellement au centre de la surface inférieure (8) et pénétrant dans la plaque intermédiaire (7), et ce de manière à tourner sur l'axe longitudinal (18);
**caractérisé en ce que**
D) le tourillon (4) comprend un trou (5) avec un axe central (6) qui s'étend essentiellement de manière parallèle à la face supérieure (3);
E) dans la plaque intermédiaire (7) est ménagé un autre trou (10) avec un axe central (11) qui, lorsque le composant est assemblé, peut être orienté de manière coaxiale par rapport à l'axe central (6);
F) le composant comprend, en outre, un élément d'assemblage (29) qui peut être introduit dans les deux trous (5;10); et
G) au moins un des deux trous (5;10) est réalisé, au moins sur une partie de sa longueur, sous forme de trou oblong (17) avec, parallèlement à l'axe longitudinal (18), une hauteur h et, perpendiculairement audit axe, une largeur b, de sorte que, lorsque le composant est assemblé, la plaque intermédiaire (7) peut tourner sur le tourillon (4) à l'intérieur d'un angle défini.

2. Composant selon la revendication 1, **caractérisé en ce que** la tige (13) présente un diamètre d, que h ≥ d et que la largeur maximale b du trou oblong (17) est dimensionnée de telle sorte que, lorsque le composant est assemblé, le mouvement de la tige (13) dans le trou (5;10) permet une rotation de la plaque intermédiaire (7) sur l'axe longitudinal (18) pouvant aller jusqu'à un angle de rotation maximal de ± 40°.

3. Composant selon la revendication 2, **caractérisé en ce que** l'angle de rotation maximal est de ± 10°.

4. Composant selon l'une des revendications 1 à 3, **caractérisé en ce que** b > h.

5. Composant selon l'une des revendications 2 à 4, **caractérisé en ce que** d ≤ h ≤ 2 d.

6. Composant selon l'une des revendications 1 à 5, **caractérisé en ce que** l'élément d'ancrage (14) et la plaque intermédiaire (7) présentent une section essentiellement ovale s'étendant perpendiculairement à l'axe longitudinal (18), **en ce que** l'axe central (6) est disposé essentiellement de manière parallèle à l'axe plus court de la section ovale, et **en ce que** les surfaces de glissement (15) sont situées en regard l'une de l'autre par rapport à l'axe central (6).

7. Composant selon l'une des revendications 1 à 6, **caractérisé en ce que** le tourillon (4) présente une surface latérale (21), **en ce que** le trou oblong (17) pénètre cette surface latérales aux points de pénétration (19;20), et **en ce que** la largeur b du trou oblong (17) ménagé dans le tourillon va en s'amincissant vers le centre du tourillon (4) à partir des points de pénétration (19;20).

8. Composant selon l'une des revendications 1 à 7, **caractérisé en ce que** le trou (5) ménagé dans le tourillon (4) est réalisé en tant que trou oblong (17) de forme cylindrique ou prismatique.

9. Composant selon l'une des revendications 1 à 7, **caractérisé en ce que** le trou (10) ménagé dans la plaque intermédiaire (7) est réalisé en tant que trou oblong (17) de forme cylindrique ou prismatique.

10. Composant selon l'une des revendications 1 à 9, **caractérisé en ce que** le trou oblong (17) présente une largeur minimale bₘᵢₙ et **en ce que** le diamètre d de la tige (13), la hauteur h et la largeur minimale bₘᵢₙ du trou oblong (17) sont dimensionnés de telle sorte que la tige (13) logée dans le trou (5;10) qui est réalisé sous forme de trou oblong (17), a du jeu.

11. Composant selon l'une des revendications 1 à 10, **caractérisé en ce que** le trou (12) est un trou oblong.

12. Composant selon l'une des revendications 1 à 11, **caractérisé en ce que** le tourillon (4) est de forme prismatique.

13. Composant selon l'une des revendications 1 à 11, **caractérisé en ce que** le tourillon (4) est de forme cylindrique.

14. Composant selon la revendication 13, **caractérisé en ce que** le tourillon (4) est de forme cylindrique circulaire.

15. Composant selon l'une des revendications 1 à 14, **caractérisé en ce que** l'élément d'assemblage (29) est une vis.

16. Composant selon l'une des revendications 1 à 14, **caractérisé en ce que** l'élément d'assemblage (29) est une tige (13).

17. Composant selon la revendication 16, **caractérisé en ce que** la tige (13) est protégée contre tout déplacement axial.

18. Composant selon la revendication 17, **caractérisé en ce que** le trou (10) ménagé dans la plaque intermédiaire (7) est fermé d'un côté.

19. Composant selon la revendication 17 ou 18, **caractérisé en ce que** la tige (13) est pourvue de dents disposées sur sa circonférence.

20. Composant selon la revendication 17 ou 18, **caractérisé en ce que** la tige (13) est pourvue de crochets.

21. Composant selon la revendication 17 ou 18, **caractérisé en ce que** la tige (13) est pourvue d'au moins une encoche planaire, et **en ce que** le trou (10) présente des bosses sortantes correspondantes.

22. Composant selon la revendication 17 ou 18, **caractérisé en ce que** le trou (10) est pourvu d'au moins une encoche, et **en ce que** la tige (13) présente des bosses sortantes correspondantes.

23. Composant selon l'une des revendications 16 à 22, **caractérisé en ce que** la tige (13) comprend, à une extrémité, un alésage taraudé destiné à la fixation d'un instrument.

24. Composant selon la revendication 23, **caractérisé en ce qu'**un alésage taraudé est ménagé à l'extrémité antérieure de la tige (13).

25. Composant selon l'une des revendications 14 à 24, **caractérisé en ce que** le tourillon (4) présente un diamètre D compris entre 8 et 15 mm.

26. Composant selon l'une des revendications 16 à 25, **caractérisé en ce que** la tige (13) présente un diamètre d compris entre 2 et 6 mm.

27. Composant selon l'une des revendications 1 à 26, **caractérisé en ce que** le trou (5) traverse le tourillon (4).
